# EUROPEAN PATENT SPECIFICATION

(11) **EP 4 011 304 B1**
(45) Date of publication and mention of the grant of the patent: **01.01.2025**
(21) Application number: 20851172.5
(22) Date of filing: 30.04.2020
(51) Int. Cl.: A61B 17/32, A61B 17/22, A61B 90/30

(54) **DEVICE FOR TREATING HEART VALVE OR VASCULAR CALCIFICATION**
VORRICHTUNG ZUR BEHANDLUNG VON HERZKLAPPEN- ODER GEFÄSSKALZIFIZIERUNG
DISPOSITIF POUR TRAITER UNE VALVE CARDIAQUE OU UNE CALCIFICATION VASCULAIRE

(30) Priority: 06.08.2019 CN 201910720924
(43) Date of publication of application: 15.06.2022
(73) Proprietor: SMARTWAVE MEDICAL HK LIMITED, Queensway, Admiralty (HK)
(72) Inventor: TAN, Jian Fong, Suzhou, Jiangsu 215000 (CN); ZHANG, Yi, Suzhou, Jiangsu 215000 (CN); PAN, Kongrong Karl, Suzhou, Jiangsu 215000 (CN); GUO, Ke, Suzhou, Jiangsu 215000 (CN)
(74) Representative: Engelhard, Markus
(86) International application number: PCT/CN2020/088393
(87) International publication number: WO 2021/022849

(56) References cited:
- WO-A1-2019/034778
- WO-A1-2019/034778
- CN-A- 104 519 809
- CN-A- 109 223 100
- CN-A- 110 604 607
- US-A- 5 863 284
- US-A- 6 117 064
- US-A1- 2012 209 375
- US-A1- 2014 046 353
- US-A1- 2016 135 828
- US-A1- 2016 135 828

## Description

### Technical Field

The present invention belongs to medical technology, and specifically relates to a device for treating heart valve or vascular calcification.

### Background of the Invention

Heart valve calcification is a major pathological manifestation of heart valve stenosis and regurgitation, which usually occurs in the elderly. Vascular calcification is a common pathological manifestation of atherosclerosis, hypertension, diabetic angiopathy, vascular injury, chronic kidney disease and senility.

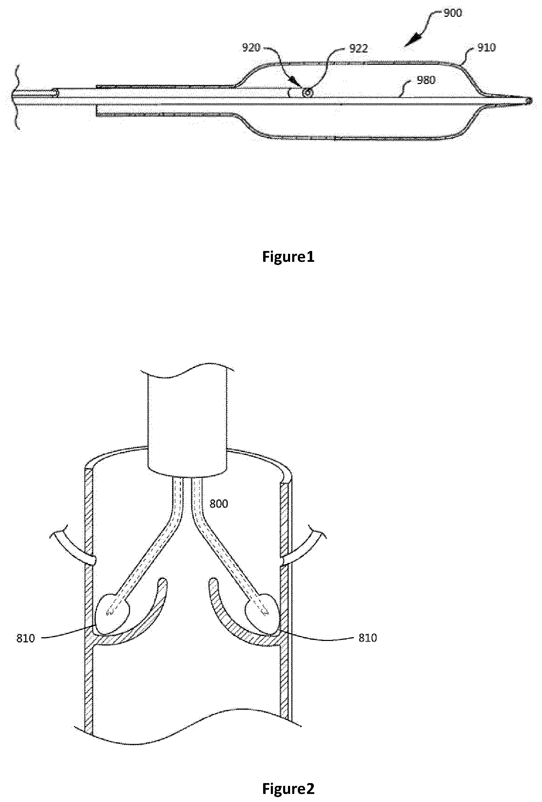

At present, shockwave balloon technology has been used to treat heart valve or vascular calcification due to its ease of operation and balloon pre-expansion. As shown in Fig. 1, a shockwave device 900 for treating heart valve calcification in prior art includes a shockwave generator 920 and a balloon 910. The shockwave generator 920 includes an electrode cable for receiving and transmitting electrical voltage/electrical current pulses and an electrode probe 922 for receiving electrical voltage/electrical current pulses to generate shockwaves, the electrode probe 922 is electrically connected to the electrode cable. The balloon 910 enfolds periphery of the shockwave generator 920, and has properties such as scalability, foldability and insulativity. The balloon 910 also has a through hole for liquid to flow into the balloon, so that the inside of the balloon 910 is filled with liquid. When internal space of the balloon 910 is filled with liquid, the balloon 910 is inflated so that at least part of the outer surface of the balloon 910 is in contact with heart valves or blood vessels with calcification (hereinafter sometimes referred to as "calcified heart valves and blood vessels" or "Calcified lesions"). The shockwave generated by the shockwave generator 920 is radially transmitted to the surface of the balloon 910 via the liquid, and then further transmitted to the calcified lesions via the surface of the balloon. When the shock wave is transmitted to the calcified lesions, the calcified tissues in the calcified lesions are fractured due to the compression stress of the shock wave. The shockwave possess the proper intensity to destroy the calcified tissues without causing additional damage to the soft tissues surrounding the calcified tissues.

However, the intensity of the shockwave is rapidly attenuated as the transmitting distance increases during the radial transmission from the electrode 922. Especially in tissues with a larger inner diameter such as the mitral or tricuspid valve, the intensity of the shock wave is attenuated drastically when the shockwave is transmitted from the electrode 922 in the center of the balloon 910 to the outer surface of the balloon 910 which is in contact with the calcified lesions of heart valves, making it difficult to obtain the ideal therapeutic effect.

Fig. 2 shows another shockwave device 800 in prior art. As shown in Fig. 2, the shockwave device 800 includes a plurality of balloons 810, each of which is provided with a shockwave generator. During the operation, the plurality of balloons 810 can be spaced apart (disperse) from each other at a specific angle, so as to make the plurality of balloons 810 contact concave portions of the cusps, respectively. However, the operations of the shockwave device 800 shown in Fig. 2 in surgeries are complicated, and it is extremely difficult to precisely locate each balloon 810 to its respective calcified lesion. Therefore, there is a higher degree of skill needed from the operator, and the operation usually requires a longer time, increasing the patient's burden and thereby decreasing the success rate of the operation.

US 2016/135828 A1 describes a shock wave valvuloplasty device
designed for treating calcified heart valves by generating shock waves within balloons that engage valve cusps to fracture or disrupt calcifications. The device features a distal anchor capable of stabilizing the shock wave-generating balloons accurately against the heart valve tissue.

US 2014/046353 A1 describes a shock wave device designed to treat calcified heart valves through the delivery of shock waves generated by a shock wave source enclosed within one or more balloons, which are inflated with a liquid.

WO 2019/034778 describes a medical device for occluding blood vessel perforations that may occur during procedures such as cardiac lead removal. The device comprises a catheter with lumens for guidewires and fluid inflation, along with a compliant, size-adjustable occlusion balloon that can be quickly inflated to seal the perforation and reduce blood loss, potentially also featuring a hemostatic composition for improved performance.

### Summary of the Invention

The invention is defined in the independent claim and other embodiments are listed in the dependent claims. No methods are claimed per se.

The present invention provides a shockwave device for treating heart valve or vascular calcification which could be operated easily and effectively inhibit attenuation of shockwave intensity, achieving the desired effect when treating heart valve or vascular calcification.

In order to address the above technical problems, one aspect of the present disclosure provides a shockwave device for treating heart valve or vascular calcification, the shockwave device includes:
a guiding tip and a plurality of balloons, at least two balloons of the plurality of balloons are connected to the guiding tip, wherein
at least one balloon of the plurality of balloons includes:
   at least one balloon body;
   at least one through hole, liquid for transmitting shock waves is filled into the balloon via the through hole to inflate the balloon; and
   at least one shockwave generator for receiving electrical voltage/electrical current pulses to generate shockwaves, the shockwave generator includes at least one electrode cable and at least one electrode probe.

The shockwave device according to one aspect of the present disclosure, wherein the guiding tip is provided at a distal end of the shockwave device, and the distal ends of all the plurality of balloons are connected to the guiding tip.

The shockwave device according to the present invention, further includes at least one inflatable component,
the inflatable component includes at least one main body and at least one through hole, fluid is filled into the inflatable component via the through hole to inflate the inflatable component, and
the plurality of balloons are distributed around periphery of the inflatable component.

The shockwave device according to one aspect of the present invention, the inflatable component has a diameter of 6-12 mm.

The shockwave device according to one aspect of the present invention, further includes at least one core wire provided inside at least one balloon body of each balloon and extending in an entire lengthwise direction of the at least one balloon body, and
the electrode probes of the shockwave generators are fixed to the core wires.

The shockwave device according to one aspect of the present invention, wherein the electrode probe includes an inner electrode and an outer electrode composed of a conductor, the inner electrode and the outer electrode are coaxially arranged and insulated from each other.

The shockwave device according to one aspect of the present invention, the inner electrode and the outer electrode are provided on periphery of the core wire in a manner of being coaxial with the core wire.

The shockwave device according to one aspect of the present invention, further includes at least one radiopaque device; the radiopaque device includes radiopaque pieces provided on at least one of the electrode probe, ends of the balloon and the core wire.

The shockwave device according to one aspect of the present invention, wherein each core wires is provided with the radiopaque pieces, and the radiopaque pieces arranged on different core wires have unique positions, shapes, lengths or numbers.

The shockwave device according to one aspect of the present invention, further includes a plurality of conductive wires, wherein each conductive wire of the plurality of conductive wires is respectively connected to at least one electrode cable to transmit electrical voltage/electrical current pulses to the shockwave generator.

The shockwave device according to one aspect of the present invention, further includes:
a delivery system connected to the through holes for allowing the liquid to flow in the delivery system and the balloons.

The shockwave device according to the one aspect of the present invention, further includes a plurality of channels in the delivery system, and
each of the plurality of channels respectively communicates with the through holes of at least one balloon.

The shockwave device according to the one aspect of the present invention, at least one channel of the plurality of channels is communicated with the through hole of the inflatable component.

The shockwave device according to the one aspect of the present invention, further includes a protective component having an umbrella-like structure that opens toward the balloons.

Another aspect according to the present disclosure provides an example method for treating heart valve or vascular calcification of animals, comprising:
delivering the shockwave device of the present invention to the targeted area;
inflating the plurality of balloons of the shockwave device so that the balloon bodies of the plurality of balloons closely contact calcified vascular wall or heart valve; and
generating shock waves by the shockwave generators to treat the calcified vascular wall or heart valve.

According to the method of one aspect of one embodiment, further comprises inflating the inflatable component of the shockwave device so that the balloon bodies of the balloons closely contact the calcified vascular wall or heart valve.

According to the method of one aspect of one embodiment, the shockwave generators of the plurality of balloons of the shockwave device generate shock waves having at least two intensities different from each other.

According to the method of one aspect of one embodiment, the shockwave generators of the plurality of balloons of the shockwave device are sequentially triggered to generate shockwaves.

According to the method of one aspect of one embodiment, at least one shockwave generator to generate shock waves having different intensities during the operation.

According to the method of one aspect of one embodiment, make the plurality of balloons and/or the at least one inflatable component have at least two inflation degrees different from each other.

According to the method of one aspect of one embodiment , at least one balloon has at least two inflation degrees different from each other during the operation.

According to the method of one aspect of one embodiment, at least one inflatable component has at least two inflation degrees different from each other during the operation.

According to the method of one aspect of one embodiment, selecting specific balloons according to at least one of the positions, the shapes, the lengths and numbers of the radiopaque pieces on the core wires, so as to control the selected balloon to have specific inflation degrees or to control shockwaves generator in the selected balloon to generate shockwaves having specific intensities.

According to the method of one aspect of one embodiment, the animal is a human.

According to an embodiment of the present invention, a shockwave device for treating heart valve or vascular calcification is provided. The shockwave device could effectively inhibit attenuation of shockwaves during transmitting and eliminate hidden dangers caused by broken balloons or leaking due to poor sealing, such that the desired treating effect could be achieved safely and reliably. Furthermore, operation of the shockwave device of this invention is easy, and the skill required by an operator decreases significantly. Thus, the operation time could be shortened, the patient's burden is decreased, the success rate of the operation is improved, and various risks that might occur during operations are decreased.

### Brief Description of the Drawings

To explain the technical solutions of this invention clearly, figures are introduced here to describe this invention's technological innovations. Apparently, the following described figures are only embodiments of the present invention, other figures could be obtained based on these figures without inventive labor for those ordinary person skilled in the art, wherein:
Fig. 1 is a structural schematic view of a shockwave device in the art;
Fig. 2 is a structural schematic view of a shockwave device in the art;
Fig. 3 is a structural schematic view of an embodiment of the shockwave device in the present invention;
Fig. 4 is a schematic view in a working state of an embodiment of the shockwave device in the present invention;
Fig. 5 is a sectional view of the balloon part of the shockwave device shown in Fig. 4.
Fig. 6 is a structural schematic view of an embodiment of the shockwave device in the present invention;
Figs. 7A and 7B are sectional views of the balloons of an embodiment of the shockwave device in the present invention;
Figs. 8A, 8B and 8C are sectional views of embodiments of the shockwave device in the present invention;
Fig. 9 is a schematic view of the conducting part of an embodiment of the shockwave device in the present invention;
Fig. 10 is a structural schematic view of an embodiment of the shockwave device in the present invention; and
Fig. 11 is a structural schematic view of an embodiment of the shockwave device in the present invention.

### Embodiments

The technical solutions this present invention can be clearly and completely described by referring to the drawings. The described embodiments are only a part of embodiments of this invention, rather than all the embodiments. All other embodiments obtained by those ordinary skilled person in the art without inventive labor, according to the embodiments of the present application belong to the protection scopes of the present application.

In the present application, the term "shockwave" is a general term of various forms of waves (such as pressure wave and the like) generated when the electrode probe discharges, rather than a limitation to a specific wave form.

In the present application, the term "distal end" of the shockwave device or components thereof indicates the end towards the guiding tip introduced into the body of the patient during an operation, while the term "proximal end" of the shockwave device or components thereof indicates the end remaining outside of the body.

In the present application, the terms "a plurality of" means two or more, and thus, the terms "a plurality of" in embodiments of the present invention could be explained as "at least two". The terms "and/or" describes the association of associated objects and represent three kinds of relationships, for example, A and/or B could represents following three situations, i.e., only A, A and B and only B. Moreover, unless otherwise defined, the term "/" generally means a relationship between two associated objects is "OR".

In the present invention, the terms "heart valve(s)" and "valve(s)" are general terms of valves including mitral valve, tricuspid valve, and aortic valve. In the present application, the terms "heart valves and blood vessels with calcification are referred to as "calcified heart valves and blood vessels" or "calcified lesions".

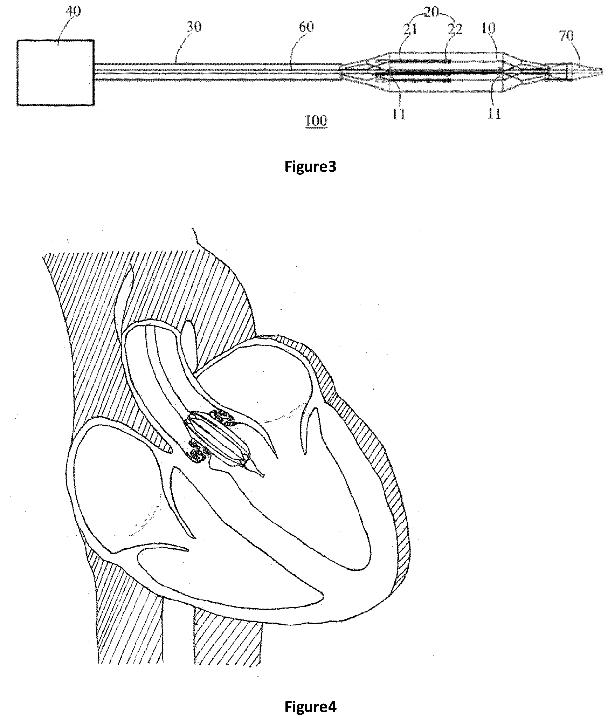

As shown in Fig. 3, the shockwave device 100 of one embodiment of the present invention includes a plurality of balloons 10. At least one balloon of the plurality of balloons 10 has at least one balloon body. The inflated balloon body of the balloon 10 shows would preferably show a cylindrical shape. There is not any specific limitation to shapes of two end parts of the balloons 10 in a lengthwise direction, as long as the balloon body of the inflated balloon 10 is cylindrical after being inflated. More preferably, balloon bodies of balloons 10 would be parallel to each other in the lengthwise direction. Specifically, axes of the respective cylindrical balloon bodies of balloons 10 in the lengthwise direction are parallel to each other. The balloons 10 of the shockwave device may have other shapes. For example, in an embodiment, a balloon may have a plurality of balloon bodies with the same shapes, such as a cylindrical shape, after being inflated by a liquid, and these balloon bodies communicate with each other such that the liquid may flow between these balloon bodies.

The balloons 10 may be formed as semi-compliant or incompliant balloons, and show properties such as scalability, foldability and insulativity. Materials for forming the balloons 10 include but are not limited to materials such as polyamides, polyether block amide (PEBA) or polyethylene terephthalate PET). One balloon 10 is provided with at least one through hole communicating with a connection pipe A14, which is used for filling liquid into the internal space of the balloon 10 to inflate the balloon. When the internal space of the balloon 10 is filled with liquid, the balloon 10 is inflated such that at least part of outer surface of the balloon 10 comes into contact with the calcified heart valves or blood vessel (calcified lesions).

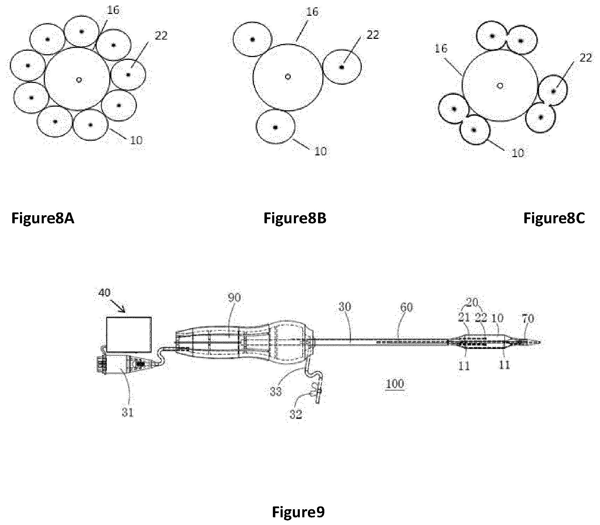

The balloon 10 is provided with at least one shockwave generator 20 in its internal space, which is used for receiving electrical voltage/electrical current pulses and generating shockwaves. Preferably, as shown in Fig. 8C, each balloon body of the balloons 10 is provided with at least one shockwave generator 20 in their internal spaces. Each shockwave generator 20 includes at least one electrode cable 21 for receiving and transmitting electrical voltage/electrical current pulses and at least one electrode probe 22 for receiving electrical voltage/electrical current pulses to generate shockwaves, the electrode probe 22 is electrically connected to the electrode cable 21. Shockwaves generated by the electrode probe 22 radially are transmitted to surfaces of the balloons 10 via the liquid, and then transmitted to the calcified lesions via the surfaces of the balloon.

As shown in Fig. 4, during an operation, the balloons 10 of the shockwave device 100 are located at the heart valves. Preferably, positions of the electrodes probes 22 in the balloons 10 are located to the calcified lesions, so as to minimize distances between the electrode probes and the calcified lesions.

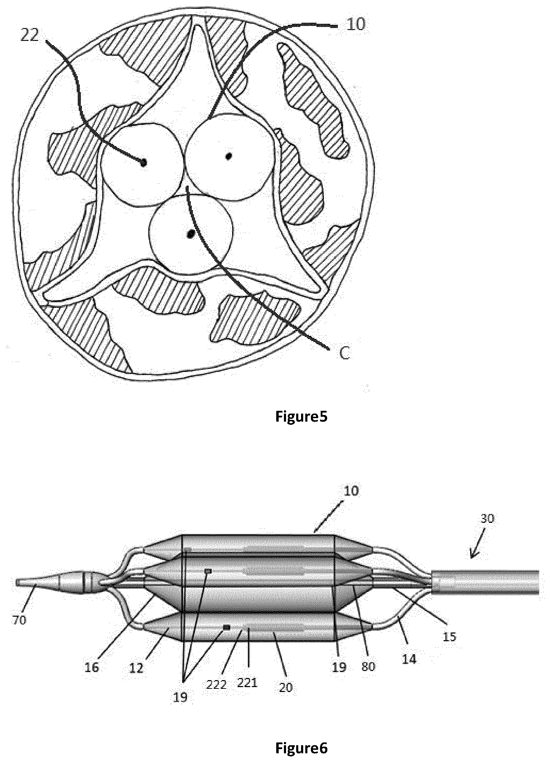

Fig. 5 shows a sectional view of the balloons part of the shockwave device 100 shown in Fig. 4 in a working state (after being inflated). As shown in Fig. 5, each balloons 10 includes a shockwave generator 20 provided in its balloon body. Therefore, compared to the shockwave device 900 in prior art shown in Fig. 1, which depicts the working state of the shockwave device 100 of one embodiment, the distances between the electrode probes 22 of the shockwave generator 20 generating shockwaves and the outer surface of the balloons 10 contacting the calcified lesions are apparently shortened. Therefore, even shockwaves generated from lower electrical voltage/electrical current pulses retain enough intensity when reaching the calcified lesions, and desired treatment effect could be obtained.

On the other hand, since the shockwave device 100 has the above mentioned configuration, i.e., cylindrical balloon bodies of each balloons 10 of the shockwave device 100 are in parallel with each other, during an operation, when the balloons contact the calcified lesions, the balloons are unlikely to be displaced. As a result, compared to the shockwave device 800 in prior art shown in Fig. 2, the shockwave device 100 of one embodiment shows an apparently decreased skill requirement from the operator. An operator with experience in general interventional surgery could expertly operate the shockwave device 100.

As a result, operation time could be shortened apparently, the patient's burden is decreased, the success rate of the operation is improved, and various risks occurred during the operation are minimized.

On the other hand, in an embodiment, since a plurality of balloons 10 are provided with blood flow intervals between the respective balloons 10, the operation could be performed while keeping the flow of blood smooth to reduce the patient's burden due to the operation. Specifically, as shown in Fig.5, there are enough intervals remained at both outer region of the balloons and inner region of the balloons, even when the plurality of balloons 10 (3 balloons in the figure) are inflated. In the embodiment shown in Fig. 5, the shockwave device 10 is provided with three balloons 10, but the number of the balloons could be two, four or even more in other embodiments.

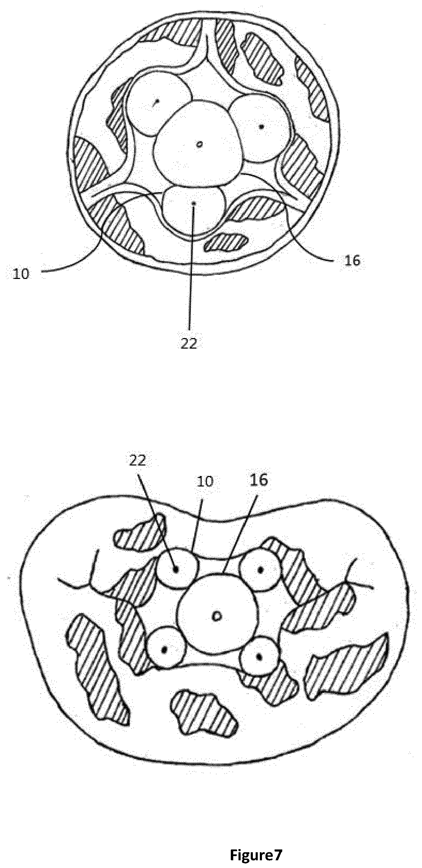

In one embodiment of the present invention, the shockwave device 100 further includes an inflatable component 16. The inflatable component 16 has at least one main body. The main body of the inflatable component 16 is preferably cylindrical after being inflated. The shapes of two end parts of the inflatable component 16 in a lengthwise direction is not specifically limited, as long as the main body of the inflated inflatable component 16 is cylindrical. As shown in Figs. 6 and 7, the inflatable component 16 is provided at the inside of the plurality of balloons 10, and in a case of the inflatable component 16 and the plurality of balloons 10 being in an inflated state, outer surfaces of the balloon 10 are closely in contact with outer surface of the inflatable component 16. There is no particular limitation of materials needed to form the inflatable component 16, for example, the inflatable component 16 may be formed as semi-compliant or incompliant balloons by using the same materials as that of the balloon 10 and shows properties such as scalability, foldability and insulativity. There is no shockwave generator provided inside the inflatable component 16. The inflatable component 16 is provided with at least one through-hole to communicat with a connection pipe B15, which is used for filling fluid into the internal space of the inflatable component 16 to inflate the inflatable component 16. The fluid used for inflating the inflatable component 16 may be the same as the liquid for inflating the balloons 10 or not, but preferably, the fluid used for inflating the inflatable component 16 is the same as the liquid for inflating the balloons 10. In the present application, there is no generic or species relationship between the terms "liquid" and "fluid", they are merely used for distinguishing materials filling and inflating the internal spaces of the balloons 10 and the inflatable component 16. According to the above configuration, since the inflatable component 16 is provided, diameters of the balloons 10 could be further decreased, and thus the distances between the electrode probes and the calcified lesions could be further shortened. Therefore, attenuation of the shockwave during transmission could be further inhibited.

In an embodiment of the present invention, the balloons 10 could be provided such that they are uniformly distributed around a periphery of the inflatable component 16. In the present application, when describing "distribute the balloons around a periphery of the inflatable component", it means the inflated balloons are distributed around periphery of the inflated inflatable component. As shown in Fig. 8A, a plurality of balloons 10 could be distributed uniformly around the periphery of the inflatable component 16.

Alternatively, as shown in Figs. 8B and 8C, a plurality of balloons could be distributed around the periphery of the inflatable component 16 at intervals. Therefore, when only a part of a patient's heart valves has calcified, the calcified lesions could be targeted specifically for treatment, and burdens to un-calcified tissues could be reduced. Moreover, as shown in Figs. 7A and 7B, by distributing a plurality of balloons 10 around the periphery of the inflatable component 16 at intervals, intervals between the balloons (including balloons 10 and the inflatable components 16) could be increased to further ensure smooth blood flow during an operation.

In an embodiment of the present invention, the shockwave device 100 may have a plurality of inflatable components 16. The plurality of inflatable components 16 are provided inside the plurality of balloons 10, and the plurality of inflatable components 16 could be provided to show regular or irregular shapes in a lengthwise directional cross section. And the plurality of balloons 10 are provided around periphery of the regular or irregular shapes of the plurality of inflatable components 16. According to the above configuration, treatment of heart valves having irregular shapes (such as mitral valve) could be performed more effectively.

Furthermore, in an embodiment of the present invention, each balloon 10 is provided with an electrode probe 22. While in other embodiments of the present invention, a plurality of electrode probes 22 could be provided in one balloon 10.

Preferably, as shown in Fig. 6, the shockwave device of the present invention also includes a core wire 12. Preferably, the core wires 12 are provided in internal spaces of each balloon body of the balloons 10 and extends and penetrates the balloon bodies in the lengthwise direction of the balloon bodies, respectively. The core wires 12 could be made from materials such as stainless steel, Ni-Ti alloy or polymer. Two end parts of a core wire 12 are joined to two end parts of a balloon from the internal space of the balloon, respectively. The core wires 12 are used for fixing the shockwave generators 20 provided in the internal space of the balloons. In an embodiment of the present invention, in a balloon 10, the electrode cable 21 and the electrode probe 22 of the shockwave generator 20 are provided along the core wire 12 and fixed to the core wire 12. In an embodiment of the present invention, the shockwave generator 20 could be fixed to the core wire 12 by means of adhesive, heat-shrinkable tubing or soldering. As shown in Fig. 6, in an embodiment of the present invention, the electrode probe 22 may include at least a pair of coaxial tips made from electrical conductors, i.e., an outer electrode 221 and an inner electrode 222. The outer electrode 221 and the inner electrode 222 are made from electrical conductors such as copper, stainless steel, or conductive polymer, and are separated by an insulation layer provided there between. In an embodiment of the present invention, the outer electrode 221 and the inner electrode 222 may have annular or arcing shapes. In an embodiment of the present invention, the outer electrode 221 and the inner electrode 222 are provided around the periphery of the core wire 12 so that they are coaxial with the core wire 12.

In the shockwave device in the present invention, a plurality of balloons 10 are provided in the inflated state, a distance from the shockwave generator 20, especially the electrode probe 22, provided in the balloons bodies of respective balloons 10 to the surfaces of the balloon is shorter. Also, in a case of providing an inflatable component 16, the distances from the electrode probes 22 to the surfaces of the balloons is further shortened. The electrode probes 22 will not contact the inner surfaces of the balloons 10, by providing the shockwave generators 20 along the core wires 12 and fixing them to the core wires 12, so as to avoid that during an operation, a balloon 10 may be damaged by sparks generated by discharging between the outer electrode 221 and the inner electrode 222 of the electrode probe 22.

In an embodiment of the present invention, to ensure the balloon 10 won't be damaged by sparks generated by discharge between the outer electrodes and the inner electrodes of the electrode probes 22, end parts of the electrodes 22 could be bent with specific angles to further shorten distances from the end parts of the electrodes to the calcified lesions, inhibiting attenuation of shockwaves.

In the shockwave device 100 of an embodiment of the present application, diameters of the balloons 10 range from 2-12 mm. In a shockwave device without an inflatable component 16, the diameters of the balloons 10 preferably range from 6-10 mm, but would more preferably be between 8-10 mm. When the diameters of the balloons are larger than 12 mm, the distances between the electrodes 22 and the calcified lesions increase, risking attenuating the intensity of the shockwave.

Lengths of the balloon bodies of the balloons 10 are 20-60 mm, such as, 20 mm, 35 mm, 40 mm, 55 mm or 60 mm. If the lengths of the balloons 10 are too long, heart tissues may be damaged during an operation, and it is difficult to turning when transmitting the shockwave during an interventional surgery. On the other hand, if the balloons are too short, it is difficult to perform a positional operation of the shockwave device during an operation.

Diameters of the main bodies of the inflatable components 16 of the shockwave device of the present invention preferably range from 6-12 mm, but would more preferably be between 8-10 mm. with the inflatable components 16, the diameters of the balloon bodies of balloons 10 range from 2-8 mm, and would preferably be between 4-6 mm.

In an embodiment of the present invention, the diameters of the plurality of balloons 10 do not necessarily have to be identical. When transmitting the same electrical voltage/electrical current pulses to the shockwave generators provided in their respective balloons, shockwaves with various intensities could be finally transmitted to the calcified lesions since the diameters of the balloons are different from each other. As a result, when calcification degrees are different in different parts of a patient's heart valves, the above configuration of the shockwave device 100 of the present invention, in a case of shockwaves with the same intensity are generated by the shockwave generators 20, shockwaves may have different intensities when reaching different parts with different calcification degrees. Specifically, during an operation, balloons with smaller diameters could be provided to parts with higher calcification degrees, so as to ensure shockwaves retain higher intensities when they reaches the calcified lesions. Likewise, balloons with larger diameters could be provided to areass with lower calcification degrees, and shockwaves will retain lower intensities when reaching the calcified lesions, and burden to patient during the operation could be further reduced.

There is no particular limitation to the liquid filled into the balloons 10 of the present invention. The liquid could be an electrolyte solution such as normal saline, or the liquid could be a non-electrolyte solution such as glycerin. Similarly, the fluid filled into the inflatable component 16 could be an electrolyte solution such as normal saline, or the fluid could be a non-electrolyte solution such as glycerin. Preferably, the liquid filled into the balloons 10 would be the same as the fluid filled into the inflatable components 16.

The balloons of the shockwave device 100 of the present invention could be designed as a disposable consumable or a reusable consumable. As a reusable consumable, disinfection should be conducted before reusing. Also, since the shockwave device 100 of the present invention has a plurality of balloons 10, when one balloon is damaged, only the damaged one should be replaced, rather than the entire shockwave device, so as to apparently reduce maintenance cost of the shockwave device.

In an embodiment of the present invention, the shockwave device 100 further includes radiopaque devices for aiding doctors to precisely position the shockwave device 100 to ensure the electrode generators 20 and/or the balloons can perform treatment at a targeted area. In an embodiment of the present invention, positions of the radiopaque devices in a patient could be observed by means of an outer developing device (such as an X-ray imaging device).

In an embodiment of the present invention, the radiopaque devices include radiopaque pieces. In an embodiment of the present invention, the radiopaque pieces could be provided at the electrode probes 22. Preferably, radiopaque materials may be contained in the electrode probes 22, so that the electrode probe 22 could be observed by means of an X-ray imaging device, helping doctors to position the shockwave device 100, which means the electrode probes 22 themselves could be used as the radiopaque pieces. In an embodiment of the present application, the radiopaque devices may include radiopaque pieces 11 provided at both end parts of the balloons 11. In an embodiment of the present invention, as shown in Fig. 6, the radiopaque devices may include a plurality of radiopaque pieces 19 provided on one core wire 12. There are no particular limitations to positions for the radiopaque pieces 19 on the core wire, it could be adjusted according to various treating positions (such as mitral valve, tricuspid valve and aortic valve). There is no particular limitation to materials used for forming the radiopaque pieces, as long as they can be developed by means of an X-ray imaging device to help doctors precisely position the shockwave device 100 and ensure the shockwave generator 20 and/or the balloons could perform treatment at a targeted area. There is no particular limitation to the shape of the radiopaque piece, it may be annular or any other shape, for example, it could be annular-designed pieces fixed to the balloons 10.

In an embodiment of the present invention, as shown in Fig. 6, radiopaque pieces 19 on core wires in different balloons are provided at various positions on respective core wires 12. For example, on a core wire 12, a radiopaque piece 19 may be provided at end parts of the core wire 12 in a lengthwise direction, or a radiopaque piece 19 may be provided at a central part of the core wire 12. Alternatively, each radiopaque pieces 19 may have various shapes, lengths and numbers from each other. For example, in an image of a developing device, radiopaque pieces 19 provided at different core wires 12 could have various shapes such as a circle, rectangle, square, or triangle. According to this configuration, if the calcification degrees of the respective parts of heart valves are different from each other, precise positions of the respective balloons 10 could be achieved by radiopaque pieces 19 provided at different positions on core wires 12 in the respective balloons 10 that have different shapes, lengths or number, so as to targeted apply different electrical voltage/electrical current pulses to shockwaves generators 20 in corresponding balloons to generate shockwaves with different intensities, and an effect of applying shockwaves with different intensities to calcified lesions having a different calcification degree could be achieved.

In an embodiment of the present invention, the radiopaque devices include a developing agent that mixed in the liquid filled into the balloons 10. The developing agent may be a common developing agent used in medical field, which could be imaged in an X-ray imaging device or an angiography equipment DAS. When a balloon is filled with liquid containing a developing agent, the angiography equipment could monitor an amount of the liquid in the balloon, and when the balloon 10 is inflated by the liquid and closely contacts the calcified lesion, no more liquid should be filled into the balloon 10. This way, the balloon 10 could come into close contact with the calcified lesion, and on the other hand, the balloon 10 could be prevented from being over-inflated by the liquid to damage heart valve or vascular walls.

In an embodiment of the present invention, as shown in Fig. 9, the shockwave device 100 of the present further includes a pulse generator 40, a delivery system 30 and conductive wires 60. The delivery system 30 includes the connection pipes A14 connected to the through holes of each of the balloons 10 and the connection pipe B15 connected to the through hole of the inflatable component 16, thus the delivery system 30 is communicated with internal spaces of the balloons 10 and the inflatable component 16 to form a sealed cavity. The delivery system 30 is provided with a first hole at a position that will not penetrate into the patient's body, since liquid could flow through the first hole to be filled into the balloon and the inflatable component 16. Under such conditions, the liquid filled into the balloons 10 and the fluid filled into the inflatable component 16 are the same liquid.

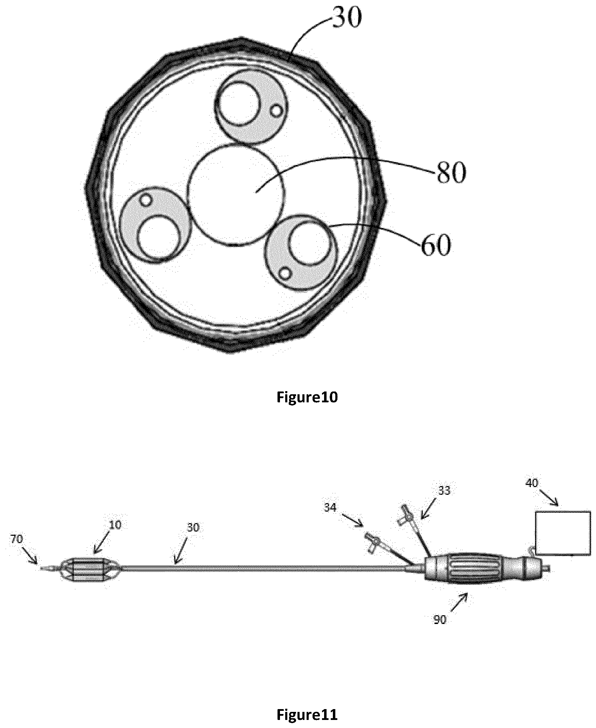

In an embodiment of the present invention, as shown in Fig. 10, a plurality of conductive wires 60 could be provided in the delivery system 30. The pulse generator 40 is connected to an outer power supply, so as to generate electrical voltage/electrical current pulses. As shown in Fig. 10, a plurality of conductive wires 60 are provided in the delivery system 30, one ends thereof are electrically connected to the pulse generator 40, and the other ends thereof are electrically connected to the electrical cables 21 of the shockwave generator 20, to transmit electrical voltage/electrical current pulses to the shockwave generators 20. In an embodiment of the present invention, one conductive wire 60 could be electrically connected to one shockwave generator 20 separately to transmit electrical voltage/electrical current pulses to each shockwave generator. Alternatively, one conductive wire of a plurality of conductive wires 60 could be electrically connected to two or more shockwave generators 20 that are provided in one balloon, or one conductive wire of the plurality of conductive wires 60 could be electrically connected to two or more shockwave generator 20 that provided in different balloons, so as to transmit electrical voltage/electrical current pulses to the two or more shockwave generators. According to the above configuration, the plurality of conductive wires 60 could be controlled by the shockwave device 100 of the present invention, respectively, to transmit electrical voltage/electrical current pulses with different intensities to shockwave generators 20 provided in different balloons 10.

During an operation, calcification may only occur at specific parts of the patient's heart valves, or calcification degrees in various parts of the patient's heart valves may be different from each other. In that case, if the same electrical voltage/electrical current pulses are transmitted to all shockwave generators 20 to generate shockwave with the same intensity, there is a risk that normal parts or parts having lower calcification degrees of the heart valves may bear unnecessary burdens. According to the shockwave device having the above configuration, electrical voltage/electrical current pulses having different intensities could be transmitted to the respective shockwave generators corresponding to the respective calcified parts of the heart valves in accordance with various calcification degrees thereof. For example, electrical voltage/electrical current pulses with higher intensities are transmitted to calcified lesions having higher calcification degrees, to generate shockwaves having higher intensities, and electrical voltage/electrical current pulses with lower intensities are transmitted to calcified lesions having lower calcification degrees, to generate shockwaves having higher intensities. As a result, shockwaves having different intensities could be applied to appropriate calcified lesions having different calcification degrees to further reduce burden to a patient.

In an embodiment of the present invention, when a part contacting one/a plurality of balloons 10 or one/a plurality of balloon bodies of one balloon 10 is not calcified, electrical voltage/electrical current pulses transmitted to the corresponding balloons/balloon bodies could be lowered to zero, to stop generating shockwave to further reduce the burden to patients.

In an embodiment of the present invention, the plurality of conductive wires 60 could be controlled separately to circularly activate the electrode probes 22 of the shockwave generators 20 of the plurality of balloons 10. In other words, the shockwave generators in the plurality of or all balloons generatting shockwaves at the same time could be prevented to further reduce burden to patient during an operation.

In an embodiment of the present invention, the above effect could be effectively achieved by selectively controlling the shockwave generators in the balloons in accordance with radiopaque pieces 10 having different positions, shapes, lengths or numbers provided on core wires 12 in balloons 10.

In an embodiment of the present invention, a plurality of channels could be provided in the delivery system 30. In an embodiment of the present invention, the channels may include the connection pipes A14 communicating with each balloons 10, and the connection pipes B15 communicating the inflatable components 16 to deliver liquid and fluid to each balloons 10 and the inflatable components 16, respectively. Moreover, each channel of the plurality of channels may be communicated with the first hole, to deliver liquid/fluid to the plurality of channels from outside. One channel of the plurality of channels could be communicated with through holes of one or more balloons 10 by connection pipes A, to deliver liquid to the balloons. At least one channel of the plurality of channels could be communicated with through holes of the inflatable components 16 by connection pipes B, to deliver fluid to the inflatable components 16. The plurality of channels are made from flexible materials, and thus have properties such as scalability, foldability and insulativity. According the above configuration, the plurality of channels may be controlled separately by the shockwave device 100 of the present invention to inflate the plurality of balloons and/or the inflatable components 16 with various inflation degrees, respectively. In the present invention, the terms "inflation degree" indicate a ratio between the volume of liquid or fluid filled into the a balloon 10/an inflatable component 16 and a max filled volume in the balloon 10/the inflatable component 16 (max liquid/fluid capacities of the balloon 10/the inflatable component 16). For example, in a balloon, when volume of liquid actually filled into the balloon 10 is the same as the max liquid capacity of the balloon 10, i.e., the balloon is completely filled with liquid and a shape of the balloon has reached a critical state (the balloon will be broken if a shape exceeds the critical state), the inflation degree of the balloon is 100%. When volume of liquid actually filled into the balloon 10 is smaller than the maximum liquid capacity of the balloon 10, the inflation degree is less than 100%. In the present invention, a lower limit of the inflation degree of the balloons should ensure the electrode probes generating shockwaves are surrounded by liquid and the electrode probes do not contact the balloon wall, and spaces between the electrode probes and the surfaces contacting the calcified lesions is filled with liquid to effectively transmit shockwaves to the calcified lesions from the electrode probes. Similarly, in the present invention, an upper limit of inflation degree of the inflatable components 16 is 100%. On the other hand, a lower limit of inflation degree of the inflatable components 16 is that the inflatable components 16 could support balloons 10 arranged around its periphery.

During an operation, a patient's heart valves will press the balloons 10, then balloons with higher inflation degrees could keep distances between electrode probes in the balloons and inner surfaces of the balloons approach or equal to radii of the balloons, respectivley. While balloons having lower inflation degrees will slightly deform, and will cause the distances between the electrode probes and inner surfaces of the balloons to be smaller than the diameters of the balloons, i.e., distances between the electrode probes and calcified lesions could be shortened, so as to further inhibit attenuation of the shockwaves. As a result, when various parts of a patient's heart valves have different calcification degrees, balloons 10 could be controlled to have various inflation degrees in accordance with various calcified lesions, an effect that applying shockwaves with different intensities to various calcified lesions could be achieved.

In an embodiment of the present invention, the above effect could be effectively achieved by selectively controlling inflation degrees of balloons according to radiopaque pieces 19 having different positions, shapes, lengths or numbers provided on core wires 12 in balloons 10.

In an embodiment of the present invention, shockwaves having various intensities could be generated by separately controlling the plurality of balloons 10 during an operation, according to actual situation of a patient; inflation degrees of the plurality of balloons and/or at least one inflatable component 16 could be separately controlled, to separately control intensities of shockwaves transmitted to calcified lesions with different calcification degree; or the above two manners could be combined, to generate/transmit shockwaves with different intensities to calcified lesions with various calcification degrees.

At the beginning of an operation, since calcification degree at the calcified lesions is higher, shockwaves with higher intensity should be applied. As the operation progresses, calcified tissues in the calcified lesions are smashed or decomposed such that the calcification degree of the calcified lesions becomes lower. At this time, the intensity of the shockwaves applied to the calcified lesions could be lowered to keep treating the calcified lesions with shockwaves having lower intensity. As a result, in an embodiment of the present invention, electrical voltage/electrical current pulses transmitted to various shockwave generators could keep being adjusted at different stages of an operation to generate shockwaves with different intensities at different stages of the operation by one shockwave generator appropriate to the treatment of the calcified lesions during the operation. Alternatively, volumes of liquid and/or fluid delivered to various balloons and/or inflatable components could be adjusted at different stages of the operation, to make the balloons have different inflation degrees at different stages of the operation, and to apply shockwaves with different intensities to the same calcified lesions at different stages in the operation. According to the above embodiment of the present invention, the burden to a patient could be further reduced.

In an embodiment of the present invention, balloons 10 corresponding to valve tissues without calcification could be kept in a compressed state by controlling a corresponding channel to stop liquid delivery to the balloon. At this time, accordingly, corresponding conductive wires 60 could be controlled to stop transmitting electrical voltage/electrical current pulses to the shockwave generator 20 in the balloon 10. According to the above configuration, an object that only treats calcified heart valves could be achieved, and thus the burden to a patient could be further reduced.

As shown in Fig. 9, in an embodiment of the invention, a channel 33 could be provided at the first hole, which is out of the delivery system 30, and be used to communicate with the first hole, or the channel 33 could extend into the delivery system 30 along the first hole from outside of the delivery system 30, and communicate with a plurality of channels in the delivery system. Similarly, liquid/fluid in balloons 10 and inflatable component 16 could flow out via the delivery system 30 (or the plurality of channels) and the first hole. Preferably, in an embodiment of the present invention, as shown in Fig. 11, a second hole may be provided on the delivery system 30 which is communicated with channels provided with the connection pipes B15 connected to the through holes of the inflatable components 16 to supply fluid into the inflatable components 16 separately or it can be used to flow the fluid out from the inflatable components 16. Preferably, a channel 34 positioned at the outer part of the delivery system 30 and communicating with the second hole could be provided at the second hole. The delivery system 30 may be made of flexible materials, and thus has properties such as scalability, foldability and insulativity. Surface of the delivery system 30 is arc-shaped, for example, the delivery system 30 shape may be globoids, such as a sphere, an ellipsoid, or a convex sphere with a curve.

In an embodiment of the present invention, the shockwave device 100 further includes a control valve 32 provided at delivering path of liquid/fluid to control the on/off of the liquid/fluid. Specifically, the control valve may be provided on the above mentioned channel 33 for easier control of the medical persons.

In an embodiment of the present application, as shown in Fig. 3, the shockwave device further includes a guiding tip 70 located at the distal end of the shockwave device 100 that is away from the delivery system 30. The guiding tip 70 functions as a guide for guiding the balloons 10 into the blood vessel or heart valve. Preferably, the guiding tip 70 has a conical shape, and a distal end of the conical shape would be smooth without sharp corners to avoid breaking the blood vessels or heart valves during an operation. Furthermore, the guiding tip 70 is made of a flexible material and thus can deform a certain amount such that it could be bent along a shape of blood vessel, allowing the shockwave device to be flexible during a delivery operation.

In an embodiment of the present invention, distal ends of at least two balloons of the plurality of balloons of the shockwave device 100 are connected to each other. Preferably, in the shockwave device 100 of an embodiment of the present invention, distal ends of all the plurality of balloons 10 are connected to the guiding tip 70. More preferably, in the shockwave device 100 of an embodiment of the present invention, all distal ends of the balloons 10 and inflatable components 16 are connected to the guiding tip 70. According to this configuration, damage to blood vessel, heart valve and heart tissue could be avoided by dispersion of the distal ends of the balloons 10 during an operation.

The shockwave device 100 according to the present invention further includes a reserved channel 80. The reserved channel 80 is located inside the shockwave device 100 and extends from a handle to the guiding tip 70 via the delivery system 30 and the balloons. During an operation, metal wires used for guiding a moving direction of the shockwave device 100 could pass through the reserved channel 80 after entering the reserved channel or other auxiliary instruments.

In the shockwave device 100 of the present invention, the reserved channel 80 could be provided at an interval C in an inside region of the plurality of balloons 10 as shown in Fig. 5. When the shockwave device of the present invention is provided with one inflatable component 16, the reserved channel 80 could be provided inside the inflatable component 16 and pass through the inflatable component 16 in a lengthwise direction of the inflatable component 16. Preferably, the reserved channel 80 may be provided in the connection pipe B15 of the inflatable component 16 to enter the inflatable component 16 via the connection pipe B15. Preferably, when the shockwave device 100 of the present invention is provided with two or more inflatable components 16, the reserved channel 80 could be provided at an interval among the two or more inflatable components 16 or could be provided in one inflatable component.

In one embodiment of the present invention, the shockwave device 100 further includes a protective umbrella. The protective umbrella is made from a hyperelastic material. The protective umbrella may be provided at a periphery of the delivery system 30, and has an open and a closed state. During an operation, a distal end of the protective umbrella is open towards the balloons 10, and a proximal end of the protective umbrella is kept at the outer surface of the delivery system 30, such that the protective umbrella 23 turns into the open state, showing an opened umbrella-like structure towards the balloons 10 from the closed state. According to the above configuration, during an operation, dissociative biological tissue fragments could be prevented from passing through by the protection umbrella, such that these biological tissue fragments will not enter blood vessel.

As shown in Fig. 9, the shockwave device 100 of one embodiment of the present invention further includes a handle, which makes the shockwave device suitable for interventional surgery. On the other hand, when a patient should be treated by a surgical operation, a handheld shockwave device could be designed. Specifically, the handle 90 could be connected to an end of the delivery system 30 at a direction away from the balloons 10. There is not any particular limitation to how the connection should be made between the handle 90 and the delivery system 30, for example, they could be connected by screw tightening or clamping.

During an operation, the handle 90 is operated by a doctor, and thus the handle is designed to an arc-shape which is suitable to be handled by a doctor. In order to decrease the possibility that the handle 90 surges, concave-convex structures could be provided to the outside of the handle 90 or increase a roughness of the outer surface of the handle 90 to increase frictional force between the handle 90 and a human hand. The handle 90 is also provided with a connector 31 electrically connected to the conductive wires 60 to connect the pulse generator, connecting the pulse generator and the shockwave generator 20.

In an embodiment, a controlling switch system is provided on the handle 90 or the pulse generator 40 to adjust the electrical current/electrical voltage pulses output intensities, repetition frequencies, and durations according to the calcification degrees of the targeted area (such as heart valves, valve leaflets and blood vessels) of a patient. Furthermore, a LED light source could be provided at the handle 90, which could be used for lighting during an operation.

The above describes the shockwave device of the present invention by referring to an example in which heart valve of a patient calcified. However, it should be understood that the above description is also suitable for treating vascular calcification of a patient.

The following will describe an example method for applying the shockwave device of the present application.

Specifically, during an operation, when using the shockwave device 100 of the present invention, balloons 10 are pushed into the body of a patient by following the guiding tip 70 with a guiding function. Positions of radiopaque devices could be observed by medical persons by an imaging device (for example, an X-ray imaging device), and then the balloons 10 of the shockwave device 100 are positioned at targeted areas.

Liquid and/or fluid is filled into the balloons 10 and/or the inflatable components 16 via the delivery system 30 to inflate the balloons 10 and/or the inflatable components 16 to make balloon bodies of the balloons 10 come into close contact with the calcified heart valves or vascular walls. Next, electrical voltage/electrical current pulses are generated by the pulse generator 40 and transmitted to the shockwave generators 20 by the conductive wires to generate shockwaves, and then the shockwaves are transmitted to the targeted area via the liquid. After treating, the liquid is flowed out from the balloons 10 and fluid is flowed out from the inflatable components 16, then the shockwave device 100 is moved away from the patient's body.

Specifically, in a case of interventional surgery, firstly, the shockwave device 100 for treating heart valve and vascular calcification is guided into the body of a patient by a delivering device via hemostatic valves along a path of an accessing device, and then is delivered to the targeted area with a help of an image device. Next, with angiography equipment DAS, normal saline containing developing agent is filled into the balloons 10 via the first hole and normal saline without developing agent is filled into the inflatable components 16, such that balloons bodies of the balloons 10 closely contact the calcified heart valve or vascular wall. After the controlling switch system is opened, parameters are adjusted, and shockwaves are generated by the shockwave generators 20 to treat the targeted area. After treating, the normal saline containing developing agent is flowed out from the balloons 10 to outside of the shockwave device via the first hole, and the normal saline without developing agent is flowed out from the inflatable components 16 to the outside of the shockwave via the second hole, so as to decompress the balloons 10 and the inflatable components 16. The shockwave device 100 is taken out from the accessing device, and the treating procedure is over. On the other hand, in a case of a surgical operation, after opening the patient's chest an operator, an incision at the apex cordis is cut, then the shockwave device 100 is guided into the heart along an accessing path build in advance, and reaches the targeted area with the help of radiopaque pieces 11; With the angiography equipment DAS, normal saline containing developing agent is filled into the balloons 10 via the first hole and normal saline without developing agent is filled into the inflatable components 16, such that balloons bodies of the balloons 10 closely contact the calcified heart valve or vascular wall; the controlling switch system is opened, parameters are adjusted, and then shockwaves are generated by the shockwave generators 20 to treat the targeted area; After treating, the normal saline containing developing agent is flowed out from the balloons 10 to outside of the shockwave device via the first hole, and the normal saline without developing agent is flowed out of the inflatable components 16 to the outside of the shockwave via the second hole, so as to decompress the balloons 10 and the inflatable component 16; the shockwave device 100 is taken out from the accessing device, and finish the treating procedure.

The above embodiments describe the configurations of the shockwave device by taking a human as a treating subject. But the subject of the shockwave device is not limited to humans; it can but also be used on animals. For example, the subjects of the shockwave device could be pets such as catsand dogs, large animals such as cows and horses, and rare wild animals such as pandas.

The above is only an embodiment of the invention and does not limit the patent scope of the invention. The invention is defined by the appended claims.

## Claims

1. A shockwave device (100) for treating heart valve or vascular
calcification, wherein
the shockwave device (100) includes:
a guiding tip (70) ;
a plurality of balloons (10), at least two balloons (10) of the plurality of balloons (10) are connected to the guiding tip (70), and
at least one inflatable component (16), wherein
at least one balloon (10) of the plurality of balloons (10) includes:
at least one balloon body;
at least one through hole, liquid for transmitting shock waves is filled into the balloon via the through hole to inflate the balloon (10); and
at least one shockwave generator (20) for receiving electrical voltage/electrical current pulses to generate shock waves, the shockwave generator (20) includes at least one electrode cable (21) and at least one electrode probe (22);
wherein the inflatable component (16) includes at least one main body and at least one through hole, fluid is filled into the inflatable component (16) via the through hole to inflate the inflatable component (16), and the plurality of balloons (10) are distributed around periphery of the inflatable component (16).

2. The shockwave device (100) according to claim 1, **characterized in that**,
the guiding tip (70) is provided at a distal end of the shockwave device (100), and distal ends of all the plurality of balloons (10) are connected to the guiding tip (70).

3. The shockwave device (100) according to claim 1 or 2, **characterized in that**, the inflatable component (16) has a diameter of 6-12mm.

4. The shockwave device (100) according to any one of claims 1 to 3, **characterized in that**, the shockwave device (100) further includes at least one core wire (12) provided in at least one balloon body of the balloons (10) and extending in an entire lengthwise direction of the at least one balloon body, and
the electrode probes (22) of the shockwave generators (20) are fixed to the core wires (12).

5. The shockwave device (100) according to any one of claims 1 to 4, **characterized in that**, wherein the electrode probe (22) includes an inner electrode (222) and an outer electrode (221) composed of a conductor, the inner electrode (222) and the outer electrode (221) are coaxially arranged and insulated from each other.

6. The shockwave device (100) according to claim 5, **characterized in that**, the inner electrode (222) and the outer electrode (221) are provided on periphery of the core wire (12) in a manner of being coaxial with the core wire (12).

7. The shockwave device (100) according to any one of claims 1 to 6, **characterized in that**, further includes at least one radiopaque device, the radiopaque device includes radiopaque pieces provided on at least one of the electrode probe (22) and ends of the balloons (10).

8. The shockwave device (100) according to any one of claims 4 to 6, **characterized in that**, further includes at least one radiopaque device, the radiopaque device includes radiopaque pieces provided on at least one of the electrode probe (22), ends of the balloons (10) and the core wire (12).

9. The shockwave device (100) according to claim 8, **characterized in that**, wherein each core wires (12) is provided with the radiopaque pieces, and the radiopaque pieces arranged on different core wires (12) have unique positions, shapes, lengths or numbers, respectively.

10. The shockwave device (100) according to any one of claims 1 to 9, **characterized in that**, the shockwave device (100) further includes a plurality of conductive wires (60), wherein each conductive wires (60) of the plurality of conductive wires (60) is respectively connected to at least one electrode cable (21) to transmit electrical voltage/electrical current pulses to the shockwave generator (20).

11. The shockwave device (100) according to any one of claims 1 to 10, **characterized in that**, further includes:
a delivery system (30) connected to the through holes for allowing the liquid to flow in the delivery system (30) and the balloons (10).

12. The shockwave device (100) according to claim 11, **characterized in that**, further includes a plurality of channels in the delivery system (30), and
each of the plurality of channels respectively communicates with the through holes of at least one balloon (10).

13. The shockwave device (100) according to claim 11, **characterized in that**, at least one channel of the plurality of channels is communicated with the through hole of the inflatable component (16).

14. The shockwave device (100) according to any one of claims 1 to 13, **characterized in that**, further includes a protective component (23) having an umbrella-like structure that opens toward the balloons (10).

## Patentansprüche

1. Stoßwellengerät (100) zum Behandeln von Herzklappen- oder Gefäßverkalkung, wobei das Stoßwellengerät (100) umfasst:
eine Führungsspitze (70);
eine Vielzahl von Ballons (10), wobei mindestens zwei Ballons (10) der Vielzahl von Ballons (10) mit der Führungsspitze (70) verbunden sind, und
mindestens eine aufblasbare Komponente (16), wobei
mindestens ein Ballon (10) der Vielzahl von Ballons (10) umfasst:
mindestens einen Ballonkörper;
mindestens ein Durchgangsloch, wobei über das Durchgangsloch Flüssigkeit zum Übertragen von Stoßwellen in den Ballon gefüllt wird, um den Ballon aufzublasen (10); und
mindestens einen Stoßwellengenerator (20) zum Empfangen von elektrischen Spannungs-/Strompulsen, um Stoßwellen zu erzeugen, wobei der Stoßwellengenerator (20) mindestens ein Elektrodenkabel (21) und mindestens eine Elektrodensonde (22) beinhaltet;
wobei die aufblasbare Komponente (16) mindestens einen Hauptkörper und mindestens ein Durchgangsloch beinhaltet, Flüssigkeit über das Durchgangsloch in die aufblasbare Komponente (16) gefüllt wird, um die aufblasbare Komponente (16) aufzublasen, und die Vielzahl von Ballons (10) um den Umfang der aufblasbaren Komponente (16) verteilt sind.

2. Stoßwellengerät (100) nach Anspruch 1, **dadurch gekennzeichnet, dass**
die Führungsspitze (70) an einem distalen Ende des Stoßwellengeräts (100) bereitgestellt ist und distale Enden all der Vielzahl von Ballons (10) mit der Führungsspitze (70) verbunden sind.

3. Das Stoßwellengerät (100) nach Anspruch 1 oder 2, **dadurch gekennzeichnet, dass** die aufblasbare Komponente (16) einen Durchmesser von 6-12 mm hat.

4. Stoßwellengerät (100) nach einem der Ansprüche 1 bis 3, **dadurch gekennzeichnet, dass** das Stoßwellengerät (100) ferner mindestens einen Kerndraht (12) beinhaltet, der in mindestens einem Ballonkörper der Ballons (10) bereitgestellt ist und sich in einer gesamten Längsrichtung des mindestens einen Ballonkörpers erstreckt, und
die Elektrodensonden (22) der Stoßwellengeneratoren (20) an den Kerndrähten (12) befestigt sind.

5. Stoßwellengerät (100) nach einem der Ansprüche 1 bis 4, **dadurch gekennzeichnet, dass** die Elektrodensonde (22) eine innere Elektrode (222) und eine äußere Elektrode (221) beinhaltet, die aus einem Leiter zusammengesetzt sind, die innere Elektrode (222) und die äußere Elektrode (221) koaxial angeordnet und voneinander isoliert sind.

6. Stoßwellengerät (100) nach Anspruch 5, **dadurch gekennzeichnet, dass** die innere Elektrode (222) und die äußere Elektrode (221) am Umfang des Kerndrahts (12) so angebracht sind, dass sie koaxial zum Kerndraht (12) verlaufen.

7. Stoßwellengerät (100) nach einem der Ansprüche 1 bis 6, **dadurch gekennzeichnet, dass** es ferner mindestens ein röntgendichtes Gerät beinhaltet, wobei das röntgendichte Gerät röntgendichte Teile beinhaltet, die an mindestens einem von der Elektrodensonde (22) und den Enden der Ballons (10) bereitgestellt sind.

8. Stoßwellengerät (100) nach einem der Ansprüche 4 bis 6, **dadurch gekennzeichnet, dass** es ferner mindestens ein röntgendichtes Gerät beinhaltet, wobei das röntgendichte Gerät röntgendichte Teile beinhaltet, die an mindestens einem von der Elektrodensonde (22), den Enden der Ballons (10) und dem Kerndraht (12) bereitgestellt sind.

9. Stoßwellengerät (100) nach Anspruch 8, **dadurch gekennzeichnet, dass** jeder Kerndraht (12) mit den röntgendichten Teilen versehen ist, und die auf verschiedenen Kerndrähten (12) angeordneten röntgendichten Teile einzigartige Positionen, Formen, Längen beziehungsweise Anzahlen aufweisen.

10. Stoßwellengerät (100) nach einem der Ansprüche 1 bis 9, **dadurch gekennzeichnet, dass** das Stoßwellengerät (100) ferner eine Vielzahl leitender Drähte (60) beinhaltet, wobei jeder leitende Draht (60) der Vielzahl leitender Drähte (60) jeweils mit mindestens einem Elektrodenkabel (21) verbunden ist, um elektrische Spannungs-/Stromimpulse an den Stoßwellengenerator (20) zu übertragen.

11. Stoßwellengerät (100) nach einem der Ansprüche 1 bis 10, **dadurch gekennzeichnet, dass** es ferner beinhaltet:
ein Verteilungssystem (30), das mit den Durchgangslöchern verbunden ist, um zu ermöglichen, dass die Flüssigkeit in das Verteilungssystem (30) und die Ballons (10) fließt.

12. Stoßwellengerät (100) nach Anspruch 11, **dadurch gekennzeichnet, dass** es ferner eine Vielzahl von Kanälen in dem Verteilungssystem (30) beinhaltet, und
jeder der Vielzahl von Kanälen jeweils mit den Durchgangslöchern von mindestens einem Ballon (10) in Verbindung steht.

13. Stoßwellengerät (100) nach Anspruch 11, **dadurch gekennzeichnet, dass** mindestens ein Kanal der Vielzahl von Kanälen mit dem Durchgangsloch der aufblasbaren Komponente (16) in Verbindung steht.

14. Stoßwellengerät (100) nach einem der Ansprüche 1 bis 13, **dadurch gekennzeichnet, dass** es ferner eine Schutzkomponente (23) mit einer schirmartigen Struktur beinhaltet, die sich zu den Ballons (10) hin öffnet.

## Revendications

1. Dispositif à ondes de choc (100) destiné à traiter une valve cardiaque ou une calcification vasculaire, dans lequel le dispositif à ondes de choc (100) inclut :
une pointe de guidage (70) ;
une pluralité de ballons (10), au moins deux ballons (10) de la pluralité de ballons (10) sont reliés à la pointe de guidage (70), et
au moins un composant gonflable (16), dans lequel
au moins un ballon (10) de la pluralité de ballons (10) inclut :
au moins un corps de ballon ;
au moins un trou traversant, un liquide destiné à transmettre des ondes de choc est introduit dans le ballon via le trou traversant pour gonfler le ballon (10) ; et
au moins un générateur d'ondes de choc (20) destiné à recevoir des impulsions de tension électrique/courant électrique pour générer des ondes de choc, le générateur d'ondes de choc (20) inclut au moins un câble d'électrode (21) et au moins une sonde-électrode (22) ;
dans lequel le composant gonflable (16) inclut au moins un corps principal et au moins un trou traversant, du fluide est introduit dans le composant gonflable (16) via le trou traversant pour gonfler le composant gonflable (16), et la pluralité de ballons (10) sont répartis autour de la périphérie du composant gonflable (16).

2. Dispositif à ondes de choc (100) selon la revendication 1, **caractérisé en ce que**
la pointe de guidage (70) est prévue à une extrémité distale du dispositif à ondes de choc (100), et les extrémités distales de l'ensemble de la pluralité de ballons (10) sont reliées à la pointe de guidage (70).

3. Dispositif à ondes de choc (100) selon la revendication 1 ou 2, **caractérisé en ce que** le composant gonflable (16) a un diamètre de 6 à 12 mm.

4. Dispositif à ondes de choc (100) selon l'une quelconque des revendications 1 à 3, **caractérisé en ce que** le dispositif à ondes de choc (100) inclut en outre au moins un fil central (12) prévu dans au moins un corps de ballon des ballons (10) et s'étendant dans toute une direction longitudinale de l'au moins un corps de ballon, et
les sondes-électrodes (22) des générateurs d'ondes de choc (20) sont fixées aux fils centraux (12).

5. Dispositif à ondes de choc (100) selon l'une quelconque des revendications 1 à 4, **caractérisé en ce que** la sonde-électrode (22) inclut une électrode interne (222) et une électrode externe (221) composées d'un conducteur, l'électrode interne (222) et l'électrode externe (221) sont agencées coaxialement et isolées l'une de l'autre.

6. Dispositif à ondes de choc (100) selon la revendication 5, **caractérisé en ce que** l'électrode interne (222) et l'électrode externe (221) sont prévues sur la périphérie du fil central (12) de manière à être coaxiales avec le fil central (12).

7. Dispositif à ondes de choc (100) selon l'une quelconque des revendications 1 à 6, **caractérisé en ce qu'**il inclut en outre au moins un dispositif radio-opaque, le dispositif radio-opaque inclut des pièces radio-opaques prévues sur au moins une des sondes-électrodes (22) et des extrémités des ballons (10).

8. Dispositif à ondes de choc (100) selon l'une quelconque des revendications 4 à 6, **caractérisé en ce qu'**il inclut en outre au moins un dispositif radio-opaque, le dispositif radio-opaque inclut des pièces radio-opaques prévues sur au moins un des sondes-électrodes (22), des extrémités des ballons (10) et du fil central (12).

9. Dispositif à ondes de choc (100) selon la revendication 8, **caractérisé en ce que** chaque fil central (12) est pourvu des pièces radio-opaques, et les pièces radio-opaques agencées sur différents fils centraux (12) ont des positions, des formes, des longueurs ou des nombres uniques, respectivement.

10. Dispositif à ondes de choc (100) selon l'une quelconque des revendications 1 à 9, **caractérisé en ce que** le dispositif à ondes de choc (100) inclut en outre une pluralité de fils conducteurs (60), dans lequel chaque fil conducteur (60) de la pluralité de fils conducteurs (60) est respectivement connecté à au moins un câble d'électrode (21) pour transmettre des impulsions de tension électrique/courant électrique au générateur d'ondes de choc (20).

11. Dispositif à ondes de choc (100) selon l'une quelconque des revendications 1 à 10, **caractérisé en ce qu'**il inclut en outre :
un système de distribution (30) relié aux trous traversants pour permettre au liquide de s'écouler dans le système de distribution (30) et les ballons (10).

12. Dispositif à ondes de choc (100) selon la revendication 11, **caractérisé en ce qu'**il inclut en outre une pluralité de canaux dans le système de distribution (30), et
chacun de la pluralité de canaux communique respectivement avec les trous traversants d'au moins un ballon (10).

13. Dispositif à ondes de choc (100) selon la revendication 11, **caractérisé en ce qu'**au moins un canal de la pluralité de canaux communique avec le trou traversant du composant gonflable (16).

14. Dispositif à ondes de choc (100) selon l'une quelconque des revendications 1 à 13, **caractérisé en ce qu'**il inclut en outre un composant de protection (23) ayant une structure en forme de parapluie qui s'ouvre vers les ballons (10).
